(19) Europäisches Patentamt
European Patent Office
Office européen des brevets

(11) EP 0 461 986 B1

(12) **FASCICULE DE BREVET EUROPEEN**

(45) Date de publication et mention de la délivrance du brevet: **10.01.1996 Bulletin 1996/02**

(21) Numéro de dépôt: **91401531.8**

(22) Date de dépôt: **11.06.1991**

(51) Int. Cl.$^6$: **C07D 295/03**, C07D 295/073, C07D 295/096, C07D 295/104, A61K 31/395

(54) **Dérivés d'hexahydroazépines, un procédé pour leur préparation et compositions pharmaceutiques les contenant**

Derivate von Hexahydroazepine, Verfahren zu ihrer Herstellung und diese enthaltende Arzneimittel

Derivatives of hexahydroazepines, procedure for their preparation and pharmaceutical compositions containing same

(84) Etats contractants désignés:
**AT BE CH DE DK ES FR GB GR IT LI LU NL SE**

(30) Priorité: **14.06.1990 FR 9007434**

(43) Date de publication de la demande:
**18.12.1991 Bulletin 1991/51**

(73) Titulaire: **SANOFI**
**F-75008 Paris (FR)**

(72) Inventeurs:
- **Lavastre, Serge**
  **F-34080 Montpellier (FR)**
- **Maignan, Jean-Pierre**
  **F-31120 Portet/Garonne (FR)**
- **Paul, Raymond**
  **F-34980 Saint Gely du Fesc (FR)**
- **Poncelet, Martine**
  **F-34270 St Mathieu de Treviers (FR)**
- **Santucci, Vincent**
  **F-34980 Combaillaux (FR)**

(74) Mandataire: **Gillard, Marie-Louise et al**
**F-75340 Paris Cédex 07 (FR)**

(56) Documents cités:
**EP-A- 0 040 744**
**EP-A- 0 163 537**
**EP-A- 0 193 875**
**DE-A- 1 545 561**
**US-A- 2 881 165**
**US-A- 4 104 383**

- **PROCEEDINGS OF THE NATIONAL ACADEMY OF SCIENCES OF THE UNITED STATES OF AMERICA, vol. 81, août 1984, pages 4983-4987; B.L. LARGENT et al.: "Psychotomimetic opiate receptors labeled and visualized with (+)-[3H]3-(3-hydroxyphenyl)-N-(1-propyl)piperidine"**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 461 986 B1

## Description

La présente invention concerne des dérivés d'hexahydroazépines se liant spécifiquement aux récepteurs sigma, notamment à ceux du système nerveux central, un procédé pour la préparation de ces composés et leur utilisation dans des compositions pharmaceutiques et plus particulièrement en tant qu'antipsychotiques.

Les récepteurs sigma ont été mis en évidence à l'aide de plusieurs ligands. En premier lieu, on peut citer un composé opiacé, le N-allylnormétazocine ou SKF-10047, plus particulièrement le composé chiral (+) SKF-10047 (W.R. Martin et al., J. Pharmacol. Exp. Ther., 1976, 197, 517-532 ; B.R.Martin et al., J. Pharmacol. Exp. Ther., 1984, 231, 539-544). Un neuroleptique, l'halopéridol, est également un ligand des récepteurs sigma, ainsi que le (+) (hydroxy-3 phényl)-3 propyl-1 pipéridine ou (+) 3-PPP (B.L. Largent et al., Proc. Nat. Acad. Sci. USA, 1984, 81, 4983-4987).

Le brevet US 4709094 décrit des dérivés de la guanidine très actifs comme ligands spécifiques des récepteurs sigma.

La distribution anatomique des récepteurs sigma dans le cerveau a été étudiée par autoradiographie, après marquage de ces récepteurs par un des ligands décrits dans le brevet US ci-dessus, à savoir la di o-tolylguanidine selon E. Weber et al., Proc. Natl. Acad. Sci. USA, 1986, 83, 8784-8788, ainsi que par les ligands (+) SKF-10047 et (+) 3-PPP selon B.L. Largent et al., J. Pharmacol. Exp. Ther., 1986, 238, 739-748. L'étude par autoradiographie a permis d'identifier nettement les récepteurs sigma du cerveau et de les distinguer des autres récepteurs des opiacés, ainsi que ceux de la phencyclidine.

Le brevet FR 2 249 659 décrit des composés de formule :

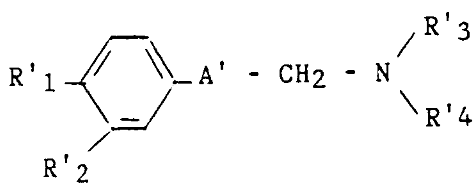
(A)

dans laquelle :

- A' représente le groupe $-CH_2-CH_2-$ ou -CH = CH- ;
- $R'_1$ représente le groupe cyclohexyle ou le groupe phényle ;
- $R'_2$ représente l'hydrogène ou un halogène ;
- $R'_3$ représente un atome d'hydrogène ou un groupe alkyle en $C_1$-$C_3$ ;
- $R'_4$ représente un groupe alkyle en $C_1$-$C_3$ ; ou $R'_3$ et $R'_4$, considérés ensemble avec l'atome d'azote auquel ils sont liés, peuvent former un groupe hétérocyclique.

Tous les produits décrits dans le document ci-dessus sont de configuration trans lorsque A = -CH=CH-. Lesdits produits sont décrits comme ayant une activité antidépressive.

La demande de brevet EP-A-040 744 décrit des composés de formule :

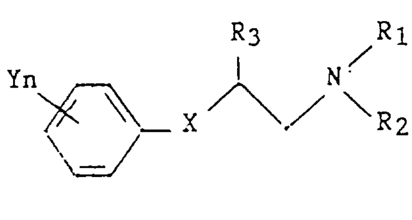
(B)

dans laquelle :

- $R_1$ et $R_2$ représentent un groupe alkyle ou constituent avec l'atome d'azote auquel ils sont liés un hétérocycle ;
- $R_3$ est l'hydrogène ou un groupe alkyle ;
- X est un groupe céto ou une oxime de formule >C=N-$OR_4$;

- Y est un groupe alkyle, un halogène, un groupe halogénoalkyle, un groupe alcoxy, un groupe alkylthio, un groupe cycloalkyle, un groupe halogénoalkylthio ou un groupe cyano ;
- n est 0, 1, 2 ou 3.

Pour ces composés (B) une activité fongicide est décrite.

La demande de brevet EP-A-0 193 875 décrit des 3-(hétéro)arylpropylamines, plus précisément leur activité fongicide.

La demande de brevet EP-A-0 163 537 décrit des dérivés 1-propanone, présentant une activité relaxante musculaire.

La demande de brevet DE-A-1 545 561 décrit un procédé pour la préparation de 1-aryl-3-aminopropines N,N-disubstituées, pharmaceutiquement actives.

Il a été maintenant trouvé une nouvelle série de dérivés de l'hexahydroazépine qui n'ont pas d'activité antidépressive et qui possèdent des propriétés inattendues et surprenantes d'antipsychotiques se liant sélectivement aux récepteurs sigma et dépourvus d'affinité pour les récepteurs dopaminergiques.

Ainsi, la présente invention se rapporte à de nouvelles hexahydroazépines N-substituées de formule :

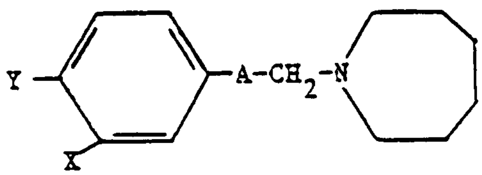

(I)

dans laquelle :

- A est un groupe choisi parmi les suivants : $-CO-CH_2-$ ; $-CH(OH)-CH_2-$ ; -CH=CH-(cis) ; $-C\equiv C-$ ;
- X représente l'hydrogène ou un halogène ;
- Y est un groupe cyclohexyle ou lorsque X est l'hydrogène, un groupe phényle; à la condition que :

  . lorsque Y est un groupe cyclohexyle et X est l'hydrogène, A soit différent de $-CH(OH)-CH_2-$ ;
  . lorsque Y est un groupe phényle, A soit différent de $-CH(OH)-CH_2-$ou $-CO-CH_2-$,

ainsi qu'à leurs sels avec des acides minéraux ou organiques.

Selon l'invention, par atome d'halogène, on entend les atomes de fluor, de chlore, de brome ou d'iode ; l'atome de chlore étant le préféré.

Lorsque A représente un groupement hydroxyéthylène, les composés (I) présentent un atome de carbone asymétrique. Les racémates ainsi que les isomères optiquement actifs de ces composés font partie intégrante de l'invention.

Les sels des composés de formule (I) selon la présente invention comprennent aussi bien ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, que ceux qui forment des sels pharmaceutiquement acceptables tels que le chlorhydrate, le bromhydrate, le sulfate, l'hydrogénosulfate, le dihydrogénophoshate, le méthanesulfonate, l'acétate, le benzoate, le citrate, le glutamate, le méthylsulfate, le maléate, le fumarate, le naphtalène-2 sulfonate.

La présente invention a également pour objets:

. des compositions pharmaceutiques contenant en tant que principe actif, un dérivé d'hexahydroazépine de formule :

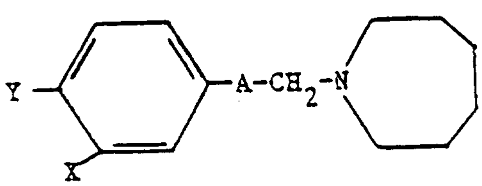

(I)

dans laquelle :

- A est un groupe choisi parmi les suivants : $-CO-CH_2-$ ; $-CH(OH)-CH_2-$ ; -CH=CH-(cis) ; -C≡C- ;
- X représente l'hydrogène ou un halogène ;
- Y est un groupe cyclohexyle ou lorsque X est l'hydrogène, un groupe phényle ;

ou un de ses sels avec des acides minéraux ou organiques ;
et

. l'utilisation d'au moins un dérivé d'hexahydroazépine de formule :

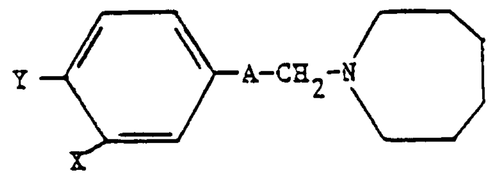

(I)

dans laquelle :

- A représente un groupe $-CH_2-CH_2-$ ou un groupe -CH=CH-(trans)
- X représente l'hydrogène ou un halogène,
- Y est un groupe cyclohexyle ou lorsque X est l'hydrogène, un groupe phényle ;

ou d'au moins un de ses sels avec des acides minéraux ou organiques,
pour la préparation de compositions pharmaceutiques utiles au traitement des troubles psychotiques.

Le chlorhydrate de cis(hexahydroazépine-1-yl)-3 (chloro-3 cyclohexyl-4 phényl)-1 propène-1 est particulièrement préféré.

La présente invention a également pour objet un procédé pour la préparation des composés (I) nouveaux, caractérisé en ce que :

a) on effectue une réaction de condensation avec le formaldéhyde et l'hexahydroazépine,

- soit sur l'acétophénone de formule :

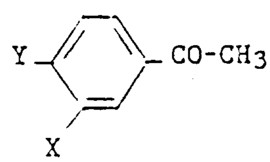

(II)

dans laquelle X et Y ont les significations indiquées ci-dessus pour (I), pour obtenir un composé (I) selon l'invention dans lequel A représente le groupement $-CO-CH_2-$,
- soit sur un dérivé phénylacétylénique de formule :

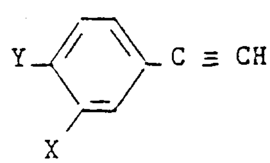

(III)

dans laquelle X et Y ont les significations indiquées ci-dessus, pour obtenir un composé (I) selon l'invention dans lequel A représente le groupement -C ≡ C- ;

b) éventuellement, on fait agir un agent réducteur sur le composé (I). dans lequel A représente un groupement -CO-$CH_2$- pour préparer le composé (I) selon l'invention dans lequel A représente un groupement -CHOH-$CH_2$- ;

c) éventuellement, on effectue une hydrogénation par l'hydrogène naissant du composé (I) dans lequel A représente le groupement acétylénique -C≡C- et on sépare les isomères cis et trans obtenus pour préparer le composé (I) dans lequel A représente le groupe -CH=CH-(cis), ou on effectue une hydrogénation en présence d'un catalyseur métallique sur support pour préparer le composé vinylénique (I) sous forme cis;

d) enfin, si nécessaire, on prépare un sel d'addition d'un composé (I) par addition d'un acide minéral ou organique approprié.

La préparation des composés de formule (I) dans laquelle A représente le groupe -CH=CH-(trans) ou le groupe -$CH_2$-$CH_2$- - composés décrits dans FR 2 249 659 et dont on revendique présentement une seconde utilisation thérapeutique - peut s'inscrire dans le procédé ci-dessus.

Les composés vinyliques sous forme trans peuvent être obtenus par hydrogénation par l'hydrogène naissant des composés acétyléniques correspondants ou par déshydratation - par exemple, avec l'acide p-toluènesulfonique dans le toluène, à la température de reflux du mélange réactionnel - des composés correspondants de formule (I) dans laquelle A=-CHOH-$CH_2$-.

Les composés de formule (I) dans laquelle A=-$CH_2$-$CH_2$- peuvent être obtenus par hydrogénation, éventuellement en présence d'un catalyseur, tel l'oxyde de platine, des composés correspondants acétyléniques ou éthyléniques.

Les acétophénones de départ (II) sont connues ou sont préparées selon des méthodes connues telles que celles décrites dans Gazz Chim. Ital. 1949, Tome 79, 453-457 et J. Am. Chem. Soc. 1947, Tome 69, 1651-1652.

Lorsque la condensation de l'étape a) du procédé selon l'invention est effectuée sur l'acétophénone (II), on procède en milieu acide dans un solvant comme par exemple l'éthanol ou le diméthoxyéthane.

On peut notamment obtenir le dérivé phénylacétylénique (III) à partir de l'acétophénone (II) en préparant un dérivé chlorophényléthylénique de formule

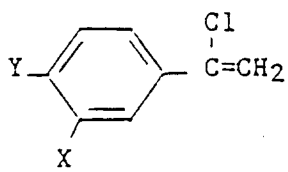

(IV)

par action du pentachlorure de phosphore sur l'acétophénone (II) et hydrolyse puis en effectuant une déshydrohalogénation du composé (IV) en milieu basique.

A partir de l'acétophénone (II), on peut également préparer une semicarbazone intermédiaire (V), puis appliquer le mode opératoire décrit par I. LALEZARI et al. (Angew. Chem . Internat. Ed., 1970, 9 (6) 464) en faisant agir l'oxyde de sélénium, à chaud, en milieu acide, puis en décomposant à chaud le sélénodiazole intermédiaire (VI) formé et obtenir ainsi le dérivé phénylacétylénique (III) selon le schéma réactionnel suivant :

(II)

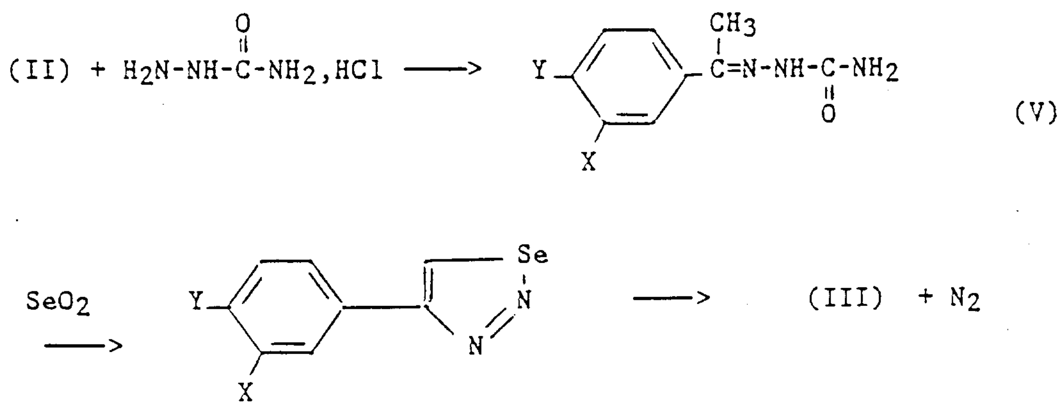


(V)

(III) + $N_2$

(VI)

Lorsque l'étape a) du procédé selon l'invention est effectuée sur le dérivé phénylacétylénique (III), on procède à chaud, dans un solvant inerte tel que le dioxanne ou le diméthoxyéthane ; pour faciliter la réaction de condensation on peut utiliser comme catalyseur un sel métallique tel que le chlorure cuivreux ou le chlorure cuivrique.

A l'étape b) du procédé, l'agent réducteur est de préférence un hydrure métallique tel que le borohydrure de sodium par exemple, et la réaction est préférentiellement réalisée dans un solvant alcoolique à une température inférieure à 10°C.

A l'étape c) du procédé : 1) l'hydrogénation par l'hydrogène naissant peut être effectuée par action du zinc dans l'acide acétique ; ou 2) lorsque l'on effectue l'hydrogénation en présence d'un catalyseur métallique sur support tel que le palladium sur sulfate de baryum ou sur carbonate de calcium, ou le nickel de Raney, dans un solvant alcoolique ou contenant une partie d'alcool, on peut opérer en présence de quinoléine pour faciliter la réaction ;l'hydrogénation catalytique ainsi réalisée conduit uniquement à des composés (I) de configuration cis (Catalytic Hydrogenation - R.L. Augustine - New-York : Marcel Dekker, 1965, p 69-71).

Le produit de formule (I) est isolé, sous forme de base libre ou de sel, selon les techniques conventionnelles.

Lorsque le composé de formule (I) et obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate.

A la fin de la réaction, le composé de formule (I) peut être isolé sous forme d'un de ses sels, par exemple le chlorhydrate ou l'oxalate ; dans ce cas, s'il est nécessaire, la base libre peut être préparée par neutralisation du dit sel avec une base minérale ou organique telle que l'hydroxyde de sodium ou la triéthylamine ou avec un carbonate ou bicarbonate alcalin, tel que le carbonate ou bicarbonate de sodium ou de potassium.

Les composés (I) selon l'invention et leurs sels, dans les essais de screening biochimiques et pharmacologiques, ont montré leur capacité d'interaction avec les récepteurs sigma. Ces essais ont été réalisés in-vitro sur des membranes de cerveau de rat en utilisant comme ligands, soit la $^3$H-(+)-3 PPP selon Largent et al., J. Pharmacol. Exp. Ther., 1986, 238, 739-740, soit la $^3$H-DTG selon Weber et al., Proc. Nat. Acad. Sci. USA, 1986, 83, 8784-8788 et in-vivo chez la souris avec le ligand $^3$H-3 PPP selon B. Kenneth Koe et al., European J. Pharmacol., 1989, 161, 263-266.

Les propriétés sur les récepteurs sigma des composés de la présente invention sont très surprenantes parce que les produits décrits dans le brevet FR 2 249 659 ne sont pas actifs sur ces récepteurs, ou leur activité est très modérée.

Ces mêmes composés et leurs sels se sont montrés actifs comme antagoniste dans le test prédictif d'une activité antipsychotique de l'hyperactivité induite chez la souris par la d-amphétamine selon P. Simon et al., La farmacoterapia nella schizofrenia, 1970, 49-66, Milan, Pacioni Mariotti, Pisa Ed.

Ces composés ne présentent pas, aussi bien in-vitro qu'in-vivo, d'affinité pour le(s) récepteur(s) dopaminergique(s) dans des essais réalisés selon Fields et al., Brain Research, 1987, 136, 578-584 et Leslie et al., Brain Research, 1987, 253-262.

Les composés selon l'invention s'avèrent donc être, selon ces essais biochimiques et comportementaux, des antipsychotiques potentiels sans impact direct au niveau dopaminergique.

Un composé représentatif, le composé de l'exemple 2, ci-après comparé à un antipsychotique connu, montre les propriétés suivantes :

TABLEAU I

| Binding in-vitro | | | | |
|---|---|---|---|---|
| | $CI_{50}$ nM | | $CI_{50}$ nM | |
| | DTG | 3-PPP | TCP | spiropéridol |
| Composé de l'exemple 2 | 10 | 8 | > 10 000 | ≃ 10 000 |
| Halopéridol | 43 | 41 | > 10 000 | 6 |
| $CI_{50}$ = concentration molaire nécessaire pour déplacer 50% du ligand lié spécifiquement ; DTG = di o-tolylguadinine ; 3-PPP = (+) (hydroxy-3 phényl)-3 propyl-1 pipéridine ; TCP = thiénylcyclohexylpipéridine. | | | | |

De ce tableau, il ressort que le composé de l'exemple 2 a un impact sigma supérieur à et/ou plus sélectif que celui de l'Halopéridol. In vivo, le composé de l'exemple 2 s'est avéré actif chez la souris sur le déplacement du ligand (+)-3PPP, au niveau du cerveau, ainsi que sur l'hyperactivité induite par la d-amphétamine.

Les composés selon la présente invention sont donc utiles comme médicaments, notamment pour le traitement des troubles psychotiques.

Les composés de la présente invention sont peu toxiques ; notamment, leur toxicité aigüe est compatible avec leur utilisation comme médicament. Pour une telle utilisation, on administre aux mammifères une quantité efficace d'un composé de formule (I) ou d'un des ses sels pharmaceutiquement acceptables.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Dans les compositions pharmaceutiques de la présente invention pour l'administration orale, sublinguale, sous-cutanée, intramusculaire, intraveineuse, transdermique, locale ou rectale, les ingrédients actifs peuvent être administrés sous formes unitaires d'administration, en mélange avec des supports pharmaceutiques classiques, aux animaux et au êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec de liants fondant à la température rectale, par exemple du beurre de cacao ou des polyéthylèneglycols.

Pour une admistration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif. Avantageusement, les unités de dosage contiennent de 0,5 à 1000 mg de principe actif de formule (I).

Les exemples suivants illustrent l'invention sans toutefois la limiter.

EXEMPLE 1

Chlorhydrate de (hexahydroazépine-1-yl)-1 (chloro-3 cyclohexyl-4 phényl)-3 propyne-3

A) Chloro-3 cyclohexyl-4 éthynyl-1 benzène.

On ajoute par petites fractions, en 30 minutes, 129 g de pentachlorure de phosphore à 118,3 g de chloro-3 cyclohexyl-4 acétophénone. En une heure, on chauffe jusqu'à 105°C, on maintient cette température pendant 1 heure et demie, puis à 115°C pendant à nouveau heure et demie. La gomme formée est extraite à l'éther éthylique, la phase éthérée est lavée avec NaOH à 5 %, séchée et concentrée. On obtient 107 g de chloro-3 cyclohexyl-4 α-chlorostyrène. Ce produit est mis en solution dans 450 ml d'éthanol puis chauffé à reflux pendant 24 heures en présence de 94 g de KOH. On concentre la majeure partie de l'alcool que l'on remplace par de l'eau, on extrait à l'éther éthylique, sèche et concentre pour obtenir 72,5 g de produit brut. Après distillation sous pression réduite, on obtient 41,7 g d'un liquide.

Point d'ébullition : 102-104°C sous 4 mm de mercure (=533 Pa).

B) Chlorhydrate de (hexahydroazépine-1-yl)-1 (chloro-3 cyclohexyl-4 phényl) -3 propyne-3

On agite, à température ambiante, un mélange de 9,5 g du produit obtenu précédemment selon A) et de 0,18 g de chlorure cuivreux dans 40 ml de diméthoxyéthane. On ajoute alors goutte à goutte à cette solution un mélange de 5,16 g d'hexahydroazépine et 6,28 g de formaldéhyde à 35 % dans l'eau en solution dans 40 ml de diméthoxyéthane et on chauffe ensuite le mélange réactionnel à reflux pendant 1 heure.

Les solvants sont concentrés sous vide, le résidu est repris dans une solution NaOH 5 %, extrait à l'éther, lavé à l'eau avec une solution saturée de chlorure de sodium, séchée sur $Na_2SO_4$, filtré et concentré sous vide. Le résidu est repris dans 250 ml d'acétate d'éthyle et l'addition d'éther chlorhydrique permet la préparation du chlorhydrate qui cristallise et est séparé par filtration.

m = 14,8 g

F = 198°C

EXEMPLE 2

Chlorhydrate de cis (hexahydroazépine-1-yl)-3 (chloro-3 cyclohexyl-4 phényl)-1 propène-1

On hydrogène, à pression atmosphérique et à température ambiante, 12,8 g de base libre du composé obtenu précédemment selon l'exemple 1, dans une solution de 100 ml d'acétate d'éthyle et de 5 ml de méthanol, en présence de 0,4 g de Pd à 5 % sur sulfate de baryum.

La catalyseur est séparé par filtration, le filtrat est concentré sous vide et l'huile résiduelle est chromatographiée sur gel de silice, éluant: dichlorométhane/méthanol : 98/2 à 95/5. Les fractions de produit pur sont concentrées sous vide, le résidu est repris dans 150 ml d'acétate d'éthyle et l'addition d'éther chlorhydrique permet la préparation du chlorhydrate qui est séparé par filtration.

m = 8,8 g

F = 168°C

EXEMPLE 3

Chlorhydrate de (hexahydroazépine-1-yl)-1 (chloro-3 cyclohexyl-4 benzoyl)-2 éthane

Un mélange de 50 g de cyclohexyl-4 chloro-3 acétophénone, 37,7 g de chlorhydrate d'hexahydroazepine, 8,5 g de trioxyméthylène et 50 ml d'éthanol est chauffé à reflux pendant 2 heures en présence de 0,85 ml d'acide chlorhydrique concentré. Le mélange prend en masse à chaud.

On refroidit, filtre et lave le précipité à l'éthanol. Le précipité est recristallisé dans un minimum d'éthanol. Les cristaux incolores obtenus sont lavés à l'éthanol, puis séchés.

m = 42,7 g

F = 208°C

EXEMPLE 4

Chlorhydrate de (hexahydroazépine-1-yl)-1 (chloro-3 cyclohexyl-4 phényl)-3 propanol-3

A une suspension de 30 g de composé obtenu selon l'exemple 3 dans 250ml de méthanol on ajoute 20 ml d'hydroxyde de sodium puis on refroidit à une température inférieure à + 5°C. On ajoute ensuite, en maintenant la température, 7,7 g de borohydrure de sodium par petites fractions. Le mélange est rendu homogène par addition de 100 ml de T.H.F. On laisse remonter la température et on agite pendant 12 heures. On ajoute ensuite 700 ml d'eau froide et on extrait le mélange à l'éther. La phase organique est séparée, lavée à l'eau jusqu'à neutralité, séchée et enfin concentrée sous vide. On obtient 26,9 g d'une huile dont 6,9 g sont dissous dans 200 ml d'éther isopropylique. On ajoute une solution d'acide chlorhydrique dans l'éthanol jusqu'à obtention d'un pH acide. Le précipité formé est séparé puis recristallisé dans l'éthanol. On obtient 4,8 g d'un composé incolore.

F = 198-200°C

EXEMPLE 5

Chlorhydrate de trans (hexahydroazépine-1-yl)-3 (chloro-3 cyclohexyl-4 phényl)-1 propène-1

On chauffe à reflux 20 g de base correspondant au composé obtenu selon l'exemple 4 et 16,3 g de monohydrate de l'acide p.toluène sulfonique, dans 500 ml de toluène, pendant 24 heures dans un appareil muni d'un séparateur d'eau. Le mélange est refroidi et on ajoute un mélange de 10 ml d'hydroxyde de sodium dans 300 ml d'eau. La phase organique est séparée, tandis que la phase aqueuse est extraite à l'éther. Les phases organiques jointes sont lavées à l'eau, séchées puis concentrées. On obtient 18,2 g d'une huile qui est reprise dans l'éther isopropylique et acidifiée avec une solution d'acide chlorhydrique dans l'éthanol. Le chlorhydrate précipité est filtré, lavé et enfin recristallisé dans l'acétonitrile. On obtient 10,7 g d'un composé incolore.

F = 216°C

EXEMPLE 6

Chlorhydrate de (hexahydroazépine-1-yl)-1 (chloro-3 cyclohexyl-4 phényl)-3 propane :

Une solution de 5 g du composé obtenu selon l'exemple 5 est hydrogénée sous pression ordinaire en présence de 250 mg de palladium sur charbon (5 %). Le volume théorique (304 ml) d'hydrogène est absorbé en 1/2 heure environ. On filtre alors le catalyseur et on concentre la solution à sec. Le résidu recristallisé dans l'acétonitrile fournit 3,2 g d'un composé incolore.

F = 196-197°C

## Revendications

**Revendications pour les Etats contractants suivants : DE, GB, FR, IT, NL, SE, CH, LI, BE, AT, LU, DK**

**1.** Dérivés d'hexahydroazépine de formule :

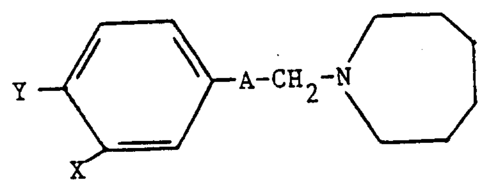

(I)

dans laquelle :

- . A est un groupe choisi parmi les suivants : $-CO-CH_2-$ ; $-CH(OH)-CH_2-$ ; -CH=CH-(cis) ; $-C\equiv C-$ ;
- . X représente l'hydrogène ou un halogène ;
- . Y est un groupe cyclohexyle ou lorsque X est l'hydrogène, un groupe phényle; à la condition que :
- . lorsque Y est un groupe cyclohexyle et X est l'hydrogène, A soit différent de $-CH(OH)-CH_2-$ ;
- . lorsque Y est un groupe phényle, A soit différent de $-CH(OH)-CH_2-$ou $-CO-CH_2-$ ;

ainsi que leurs sels avec des acides minéraux ou organiques.

**2.** Sels d'addition pharmaceutiquement acceptables d'un composé selon la revendication 1.

**3.** Chlorhydrate de cis(hexahydroazépin-1-yl)-3 (chloro-3-cyclohexyl-4-phényl)-1-propène-1.

**4.** Procédé de préparation d'un dérivé d'hexahydrozépine selon la revendication 1, de formule (I), dans laquelle A est choisi parmi les groupes $-CO-CH_2-$ et $-CH(OH)-CH_2-$, caractérisé en ce que :

a) on effectue une réaction de condensation avec le formaldéhyde et l'hexahydroazépine sur l'acétophénone de formule :

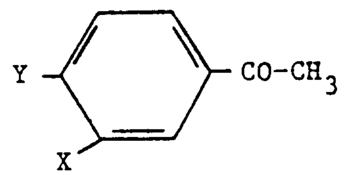

(II)

dans laquelle X et Y ont les significations indiquées à la revendication 1, pour obtenir un dérivé de formule (I) dans laquelle A représente le groupe $-CO-CH_2-$ ;

b) éventuellement, on fait réagir un agent réducteur sur le composé de formule (I) ainsi obtenu pour préparer le composé de formule (I) dans laquelle A représente le groupe $-CH(OH)-CH_2-$ ;

c) enfin, si nécessaire, on prépare un sel d'addition d'un composé de formule (I) ainsi obtenu par addition d'un acide minéral ou organique approprié.

**5.** Procédé de préparation d'un dérivé d'hexahydroazépine selon la revendication 1, de formule (I), dans laquelle A est choisi parmi les groupes -C≡C-, et -CH=CH-(cis), caractérisé en ce que :

a) on effectue une réaction de condensation avec le formaldéhyde et l'hexahydroazépine sur un dérivé phénylacétylénique de formule :

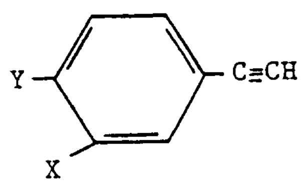

(III)

dans laquelle X et Y ont les significations indiquées à la revendication 1 pour obtenir un dérivé de formule (I) dans laquelle A représente le groupe -C≡C- ;

b) éventuellement, on prépare à partir dudit dérivé le composé vinylénique de formule (I) sous forme cis soit directement par hydrogénation en présence d'un catalyseur métallique sur support, soit par hydrogénation non sélective à l'hydrogène naissant suivie d'une séparation du mélange des isomères cis et trans obtenu ;

c) enfin, si nécessaire, on prépare un sel d'addition d'un composé de formule (I) ainsi obtenu par addition d'un acide minéral ou organique approprié.

**6.** Composition pharmaceutique contenant, en tant que principe actif, un dérivé d'hexahydroazépine de formule :

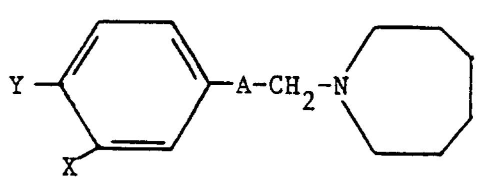

(I)

dans laquelle :

. A est un groupe choisi parmi les suivants : $-CO-CH_2-$ ; $-CH(OH)-CH_2-$ ; -CH=CH-(cis) ; -C≡C- ;
. X représente l'hydrogène ou un halogène ;
. Y est un groupe cyclohexyle ou lorsque X est l'hydrogène, un groupe phényle ;

ou un de ses sels avec des acides minéraux ou organiques.

7. Procédé selon la revendication 6, pour la préparation de compositions pharmaceutiques, sous forme d'unité de dosage.

8. Procédé selon la revendication 7, pour la préparation de compositions pharmaceutiques, contenant de 0,5 à 1 000 mg de principe actif de formule (I), par unité de dosage.

9. Utilisation d'au moins un dérivé d'hexahydroazépine de formule :

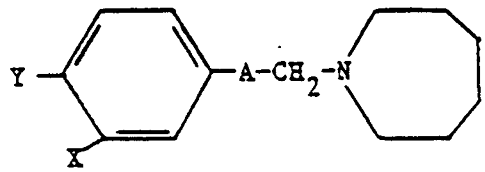

(I)

dans laquelle :

. A représente un groupe $-CH_2-CH_2-$ ou un groupe -CH=CH-(trans)
. X représente l'hydrogène ou un halogène ;
. Y est un groupe cyclohexyle ou lorsque X est l'hydrogène, un groupe phényle ;

ou d'au moins un de ses sels avec des acides minéraux ou organiques,
pour la préparation de compositions pharmaceutiques utiles au traitement des troubles psychotiques.

**Revendications pour l'Etat contractant suivant : GR**

1. Dérivés d'hexahydroazépine de formule :

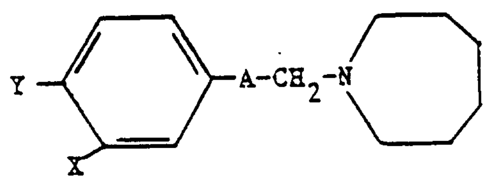

(I)

dans laquelle :

. A est un groupe choisi parmi les suivants : $-CO-CH_2-$ ; $-CH(OH)-CH_2-$ ; -CH=CH- (cis) ; -C≡C- ;
. X représente l'hydrogène ou un halogène ;
. Y est un groupe cyclohexyle ou lorsque X est l'hydrogène, un groupe phényle; à la condition que :
. lorsque Y est un groupe cyclohexyle et X est l'hydrogène, A soit différent de $-CH(OH)-CH_2-$ ;
. lorsque Y est un groupe phényle, A soit différent de $-CH(OH)-CH_2-$ou $-CO-CH_2-$ ;

ainsi que leurs sels avec des acides minéraux ou organiques.

2. Sels d'addition pharmaceutiquement acceptables d'un composé selon la revendication 1.

3. Chlorhydrate de cis(hexahydroazépin-1-yl)-3 (chloro-3-cyclohexyl-4-phényl)-1 propène-1.

4. Procédé de préparation d'un dérivé d'hexahydroazépine selon la revendication 1, de formule (I) dans laquelle A est choisi parmi les groupes $-CO-CH_2-$ et $-CH(OH)-CH_2-$, caractérisé en ce que :

a) on effectue une réaction de condensation avec le formaldéhyde et l'hexahydroazépine sur l'acétophénone de formule :

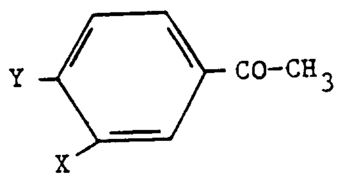

(II)

dans laquelle X et Y ont les significations indiquées à la revendication 1, pour obtenir un dérivé de formule (I) dans laquelle A représente le groupe $-CO-CH_2-$ ;
b) éventuellement, on fait réagir un agent réducteur sur le composé de formule (I) ainsi obtenu pour préparer le composé de formule (I) dans laquelle A représente le groupe $-CH(OH)-CH_2-$ ;
c) enfin, si nécessaire, on prépare un sel d'addition d'un composé de formule (I) ainsi obtenu par addition d'un acide minéral ou organique approprié.

**5.** Procédé de préparation d'un dérivé d'hexahydroazépine selon la revendication 1, de formule (I), dans laquelle A est choisi parmi les groupes -C≡C- et -CH=CH-(cis), caractérisé en ce que :

a) on effectue une réaction de condensation avec le formaldéhyde et l'hexahydroazépine sur un dérivé phénylacétylénique de formule :

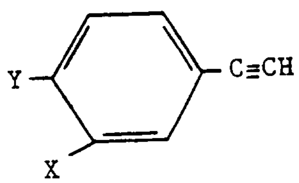

(III)

dans laquelle X et Y ont les significations indiquées à la revendication 1 pour obtenir un dérivé de formule (I) dans laquelle A représente le groupe -C≡C- ;
b) éventuellement, on prépare à partir dudit dérivé le composé vinylénique de formule (I) sous forme cis soit directement par hydrogénation en présence d'un catalyseur métallique sur support, soit par hydrogénation non sélective à l'hydrogène naissant suivie d'une séparation du mélange des isomères cis et trans obtenu ;
c) enfin, si nécessaire, on prépare un sel d'addition d'un composé de formule (I) ainsi obtenu par addition d'un acide minéral ou organique approprié.

**6.** Procédé pour la préparation de compositions pharmaceutiques, caractérisé en ce qu'il consiste à mélanger à un excipient pharmaceutique un dérivé d'hexahydroazépine de formule :

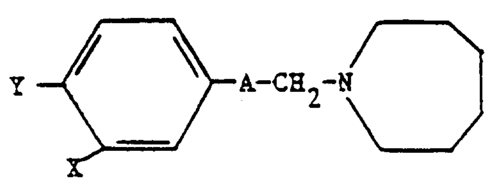

(I)

dans laquelle :

- A est un groupe choisi parmi les suivants : $-CO-CH_2-$ ; $-CH(OH)-CH_2-$ ; -CH=CH-(cis) ; -C≡C- ;
- X représente l'hydrogène ou un halogène ;
- Y est un groupe cyclohexyle ou lorsque X est l'hydrogène, un groupe phényle ;

ou un de ses sels avec des acides minéraux ou organiques.

**7.** Procédé selon la revendication pour la préparation de compositions pharmaceutiques, sous forme d'unité de dosage.

**8.** Procédé selon la revendication 7, pour la préparation de compositions pharmaceutiques, contenant de 0,5 à 1 000 mg de principe actif de formule (I), par unité de dosage.

**9.** Utilisation d'au moins un dérivé d'hexahydroazépine de formule :

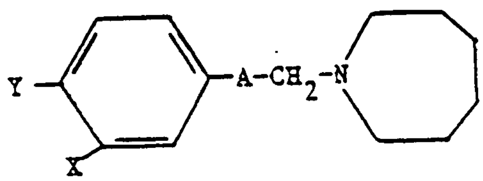

(I)

dans laquelle :

. A représente un groupe $-CH_2-CH_2-$ ou un groupe -CH=CH-(trans)
. X représente l'hydrogène ou un halogène ;
. Y est un groupe cyclohexyle ou lorsque X est l'hydrogène, un groupe phényle ;

ou d'au moins un de ses sels avec des acides minéraux ou organiques,
pour la préparation de compositions pharmaceutiques utiles au traitement des troubles psychotiques.

**Revendications pour l'Etat contractant suivant : ES**

**1.** Procédé de préparation d'un dérivé d'hexahydroazépine de formule :

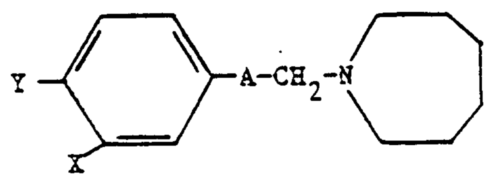

(I)

dans laquelle :

. A est un groupe choisi parmi les suivants : $-CO-CH_2-$ ; $-CH(OH)-CH_2-$ ;
. X représente l'hydrogène ou un halogène ;
. Y est un groupe cyclohexyle ;

à la condition que, lorsque X est l'hydrogène, A soit différent de $-CH(OH)-CH_2-$, ainsi que de ses sels avec des acides minéraux ou organiques ;
caractérisé en ce que :

a) on effectue une réaction de condensation avec le formaldéhyde et l'hexahydroazépine sur l'acétophénone de formule :

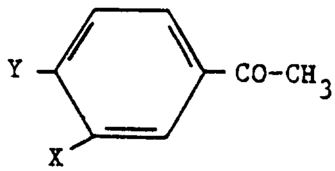

(II)

dans laquelle X et Y ont les significations indiquées ci-dessus, pour obtenir un dérivé de formule (I) dans laquelle A représente le groupe $-CO-CH_2-$ ;
b) éventuellement, on fait réagir un agent réducteur sur le composé de formule (I) ainsi obtenu pour préparer le composé de formule (I) dans laquelle A représente le groupe $-CH(OH)-CH_2-$ ;
c) enfin, si nécessaire, on prépare un sel d'addition d'un composé de formule (I) ainsi obtenu par addition d'un acide minéral ou organique approprié.

2. Procédé de préparation d'un dérivé d'hexahydroazépine de formule :

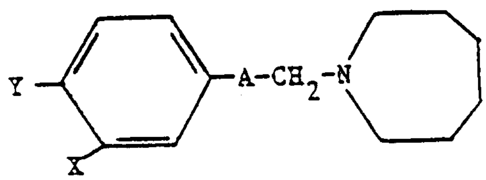

(I)

dans laquelle :

- A est un groupe choisi parmi les suivants :
  -CH=CH-(cis); -C≡C- ;
- X représente l'hydrogène ou un halogène ;
- Y est un groupe cyclohexyle ou lorsque X est l'hydrogène, un groupe phényle ;
  ainsi que de ses sels avec des acides minéraux ou organiques ;

caractérisé en ce que :

a) on effectue une réaction de condensation avec le formaldéhyde et l'hexahydroazépine sur un dérivé phénylacétylénique de formule :

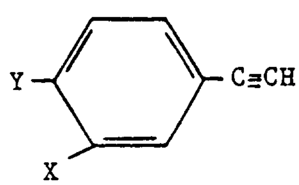

(III)

dans laquelle X et Y ont les significations indiquées ci-dessus, pour obtenir un dérivé de formule (I) dans laquelle A représente le groupe -C≡C- ;
b) éventuellement, on prépare à partir dudit dérivé le composé vinylénique de formule (I) sous forme cis soit directement par hydrogénation en présence d'un catalyseur métallique sur support, soit par hydrogénation non sélective à l'hydrogène naissant suivie d'une séparation du mélange des isomères cis et trans obtenu ;
c) enfin, si nécessaire, on prépare un sel d'addition d'un composé de formule (I) ainsi obtenu par addition d'un acide minéral ou organique approprié.

**3.** Procédé selon l'une des revendications 1 ou 2 pour la préparation d'un sel d'addition pharmaceutiquement acceptable d'un composé de formule (I).

**4.** Procédé selon la revendication 2, caractérisé en ce que :

a) on effectue une réaction de condensation avec le formaldéhyde et l'hexahydroazépine sur un dérivé phénylacétylénique de formule :

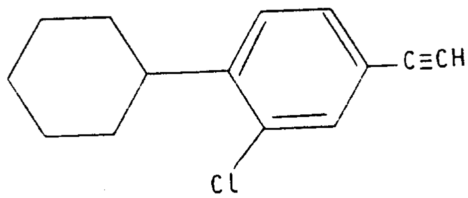

(III)

b) on effectue une hydrogénation dudit dérivé pour préparer le cis(hexahydroazépin-1-yl)-3 (chloro-3-cyclohexyl-4-phényl)-1-propène-1 ;
c) on prépare et isole ledit composé sous forme de chlorhydrate.

**5.** Procédé pour la préparation de compositions pharmaceutiques, caractérisé en ce qu'il consiste à mélanger à un excipient convenable un dérivé d'hexahydroazépine de formule :

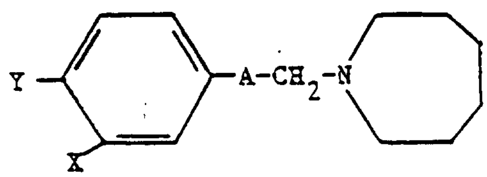

(I)

dans laquelle :

. A est un groupe choisi parmi les suivants : $-CO-CH_2-$ ; $-CH(OH)-CH_2-$ ; -CH=CH-(cis) ; -C≡C- ;
. X représente l'hydrogène ou un halogène ;
. Y est un groupe cyclohexyle ou lorsque X est l'hydrogène, un groupe phényle;

ou un de ses sels avec des acides minéraux ou organiques.

**6.** Procédé selon la revendication 5, pour la préparation de compositions pharmaceutiques sous forme d'unité de dosage.

**7.** Procédé selon la revendication pour la préparation de compositions pharmaceutiques contenant de 0,5 à 1000 mg de principe actif de formule (I), par unité de dosage.

**8.** Utilisation d'au moins un dérivé d'hexahydroazépine de formule :

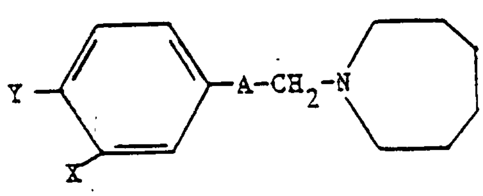

(I)

dans laquelle :

. A représente un groupe $-CH_2-CH_2-$ ou un groupe -CH=CH-(trans)
. X représente l'hydrogène ou un halogène,
. Y représente un groupe cyclohexyle ou lorsque X est l'hydrogène, un groupe phényle;

ou d'au moins un de ses sels avec des acides minéraux ou organiques, pour la préparation de compositions pharmaceutiques utiles au traitement des troubles psychotiques.

**Claims**

**Claims for the following Contracting States : DE, GB, FR, IT, NL, SE, CH, LI, BE, AT, LU, DK**

1. Hexahydroazepin derivatives of formula:

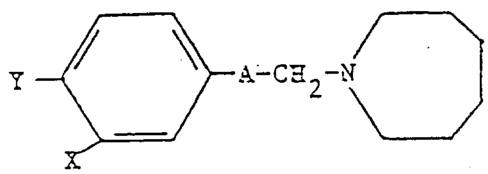

(I)

in which:

- A is a group selected from the following: $-CO-CH_2-$; $-CH(OH)-CH_2-$; -CH=CH-(cis); -C≡C-;
- X is hydrogen or a halogen atom;
- Y is a cyclohexyl group or when X is hydrogen, a phenyl group; with the proviso that:
- when Y is a cyclohexyl group and X is hydrogen, A is different from $-CH(OH)-CH_2-$;
- when Y is a phenyl group, A is different from $-CH(OH)-CH_2-$ or $-CO-CH_2-$;
as well as their salts with organic or inorganic acids.

2. Pharmaceutically acceptable addition salts of a compound according to claim 1.

3. Cis(hexahydroazepin-1-yl)-3 (chloro-3-cyclohexyl-4-phenyl)-1-propene-1 hydrochloride.

4. Method for preparing an hexahydroazepin derivative according to claim 1, of formula (I), in which A is selected from the $-CO-CH_2-$ and $-CH(OH)-CH_2-$ groups, characterized in that:

a) a condensation reaction is performed with the formaldehyde and the hexahydroazepin on the acetophenone of formula:

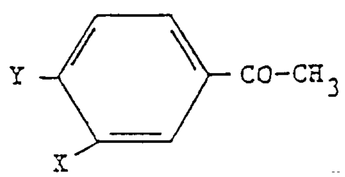

(II)

in which X and Y have the meanings indicated in claim 1, in order to obtain a derivative of formula (I) in which A is the group $-CO-CH_2-$;
b) a reducing agent is optionally reacted on the thus obtained compound of formula (I) in order to prepare the compound of formula (I) in which A is the group $-CH(OH)-CH_2-$;
c) finally, an addition salt of a thus obtained compound of formula (I) is prepared by adding an appropriate organic or inorganic acid, if necesary.

5. Method for preparing an hexahydroazepin derivative according to claim 1, of formula (I), in which A is selected from the -C≡C-, and -CH=CH-(cis) groups, characterized in that:

a) a condensation reaction is performed with the formaldehyde and the hexahydroazepin on a phenylacetylenic derivative of formula:

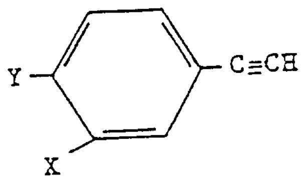

(III)

in which X and Y have the meanings indicated in claim 1, in order to obtain a derivative of formula (I) in which A is the group -C≡C-;

b) the vinylenic compound of formula (I) is optionally prepared from said derivative in the cis form either directly by hydrogenation in the presence of a supported metal catalyst or by non-selective hydrogenation with nascent hydrogen followed by a separation of the resulting mixture of cis and trans isomers;

c) finally, an addition salt of a thus obtained compound of formula (I) is prepared by adding an appropriate organic or inorganic acid, if necesary.

**6.** Pharmaceutical composition containing, as the active principle, an hexahydroazepin derivative of formula:

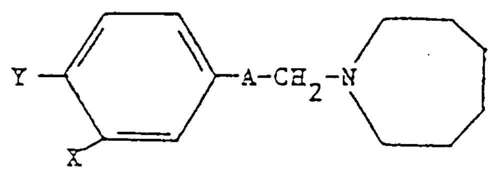

(I)

in which:

- A is a group selected from the following: $-CO-CH_2-$; $-CH(OH)-CH_2-$; -CH=CH-(cis); -C≡C-;
- X is hydrogen or a halogen atom;
- Y is a cyclohexyl group or when X is hydrogen, a phenyl group;

or one of its salts with organic or inorganic acids.

**7.** Pharmaceutical composition according to claim 6, in the form of unit doses, in which the active principle is mixed with a pharmaceutical excipient.

**8.** Pharmaceutical composition according to claim 7, containing from 0.5 to 1,000 mg of active principle of formula (I) per unit dose.

**9.** Use of at least one hexahydroazepin derivative of formula:

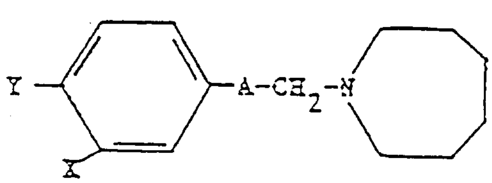

(I)

in which:

- A is a $-CH_2-CH_2-$ group or a -CH=CH-(trans) group,
- X is hydrogen or a halogen atom,
- Y is a cyclohexyl group or when X is hydrogen, a phenyl group,

or of at least one of its salts with organic or inorganic acids,
for the preparation of pharmaceutical compositions useful for the treatment of psychotic disorders.

**Claims for the following Contracting State : ES**

**1.** Method for preparing an hexahydroazepin derivative of formula:

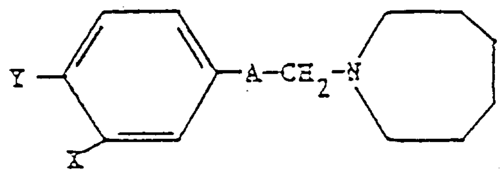

(I)

in which:

- A is a group selected from the following: $-CO-CH_2-$; $-CH(OH)-CH_2-$;
- X is hydrogen or a halogen atom;
- Y is a cyclohexyl group;

with the proviso that when X is hydrogen, A is different from $-CH(OH)-CH_2-$,
as well as its salts with organic or inorganic acids;
characterized in that:

a) a condensation reaction is performed with the formaldehyde and the hexahydroazepin on the acetophenone of formula:

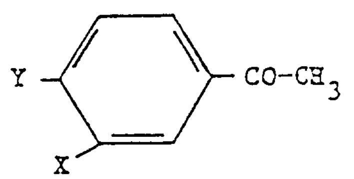

(II)

in which X and Y have the meanings indicated above, in order to obtain a derivative of formula (I) in which A is the group $-CO-CH_2-$;
b) a reducing agent is optionally reacted on the thus obtained compound of formula (I) in order to prepare the compound of formula (I) in which A is the group $-CH(OH)-CH_2$;
c) finally, an addition salt of a thus obtained compound of formula (I) is prepared by adding an appropriate organic or inorganic acid, if necesary.

**2.** Method for preparing an hexahydroazepin derivative of formula:

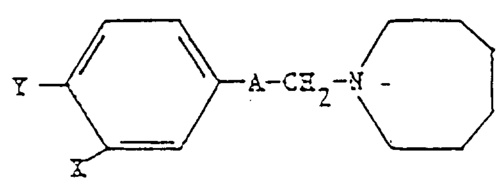

(I)

in which:

- A is a group selected from the following: -CH=CH-(cis); -C≡C-;
- X is hydrogen or a halogen atom;
- Y is a cyclohexyl group or when X is hydrogen, a phenyl group;

as well as its salts with organic or inorganic acids.
characterized in that:

a) a condensation reaction is performed with the formaldehyde and the hexahydroazepin on a phenylacetylenic derivative of formula:

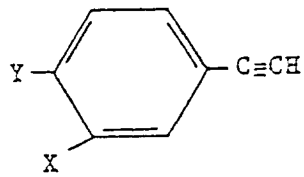

(III)

in which X and Y have the meanings indicated above, in order to obtain a derivative of formula (I) in which A is the group -C≡C-;
b) the vinylenic compound of formula (I) is optionally prepared from said derivative in the cis form either directly by hydrogenation in the presence of a supported metal catalyst or by non-selective hydrogenation with nascent hydrogen followed by a separation of the resulting mixture of cis and trans isomers;
c) finally, an addition salt of a thus obtained compound of formula (I) is prepared by adding an appropriate organic or inorganic acid, if necesary.

**3.** Method according to one of claims 1 or 2 for the preparation of a pharmaceutically acceptable addition salt of a compound of formula (I).

**4.** Method according to claim 2, characterized in that:

a) a condensation reaction is performed with the formaldehyde and the hexahydroazepin on a phenylacetylenic derivative of formula:

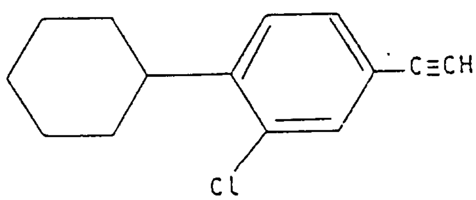

(III)

b) a hydrogenation of said derivative is performed in order to prepare the cis(hexahydroazepin-1-yl)-3 (chloro-3-cyclohexyl-4-phenyl)-1-propene-1;
c) said compound is prepared and isolated in the form of a hydrochloride.

**5.** Method for preparing pharmaceutical compositions, characterized in that it consists in mixing an hexahydroazepin derivative of formula:

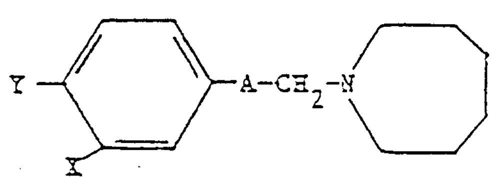

(I)

in which:

- A is a group selected from the following: $-CO-CH_2-$; $-CH(OH)-CH_2-$; -CH=CH-(cis); -C≡C-;

- X is hydrogen or a halogen atom;
- Y is a cyclohexyl group or when X is hydrogen, a phenyl group;

or one of its salts with organic or inorganic acids, with a pharmaceutical excipient.

6. Method according to claim 5, for the preparation of pharmaceutical compositions in the form of unit doses.

7. Method according to claim 6, for the preparation of pharmaceutical compositions containing from 0.5 to 1,000 mg of active principle of formula (I) per unit dose.

8. Use of at least one hexahydroazepin derivative of formula:

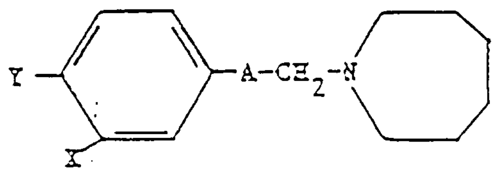

(I)

in which:

- A is a $-CH_2-CH_2-$ group or a -CH=CH-(trans) group,
- X is hydrogen or a halogen atom,
- Y is a cyclohexyl group or when X is hydrogen, a phenyl group;

or of at least one of its salts with organic or inorganic acids, for the preparation of pharmaceutical compositions useful for the treatment of psychotic disorders.

**Claims for the following Contracting State : GR**

1. Hexahydroazepin derivatives of formula:

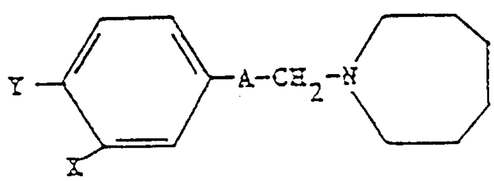

(I)

in which:

- A is a group selected from the following: $-CO-CH_2-$; $-CH(OH)-CH_2-$; -CH=CH-(cis); -C≡C-;
- X is hydrogen or a halogen atom;
- Y is a cyclohexyl group or when X is hydrogen, a phenyl group; with the proviso that:
- when Y is a cyclohexyl group and X is hydrogen, A is different from $-CH(OH)-CH_2-$;
- when Y is a phenyl group, A is different from $-CH(OH)-CH_2-$ or $-CO-CH_2-$;

  as well as their salts with organic or inorganic acids.

2. Pharmaceutically acceptable addition salts of a compound according to claim 1.

3. Cis(hexahydroazepin-1-yl)-3 (chloro-3-cyclohexyl-4-phenyl)-1-propene-1 hydrochloride.

4. Method for preparing an hexahydroazepin derivative according to claim 1, of formula (1), in which A is selected from the $-CO-CH_2-$ and $-CH(OH)-CH_2-$ groups, characterized in that:

a) a condensation reaction is performed with the formaldehyde and the hexahydroazepin on the acetophenone of formula:

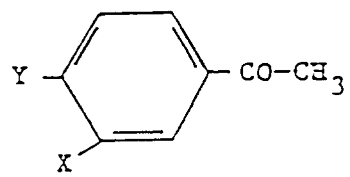

(II)

in which X and Y have the meanings indicated in claim 1, in order to obtain a derivative of formula (I) in which A is the group $-CO-CH_2-$;

b) a reducing agent is optionally reacted on the thus obtained compound of formula (I) in order to prepare the compound of formula (I) in which A is the group $-CH(OH)-CH_2-$;

c) finally, an addition salt of a thus obtained compound of formula (I) is prepared by adding an appropriate organic or inorganic acid, if necessary.

**5.** Method for preparing an hexahydroazepin derivative according to claim 1, of formula (I), in which A is selected from the -C≡C-, and -CH=CH-(cis) groups, characterized in that:

a) a condensation reaction is performed with the formaldehyde and the hexahydroazepin on a phenylacetylenic derivative of formula:

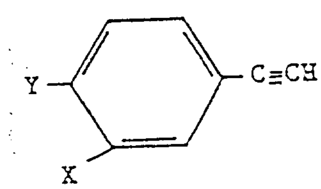

(III)

in which X and Y have the meanings indicated in claim 1, in order to obtain a derivative of formula (I) in which A is the group -C≡C-;

b) the vinylenic compound of formula (I) is optionally prepared from said derivative in the cis form either directly by hydrogenation in the presence of a supported metal catalyst or by non-selective hydrogenation with nascent hydrogen followed by a separation of the resulting mixture of cis and trans isomers;

c) finally, an addition salt of a thus obtained compound of formula (I) is prepared by adding an appropriate organic or inorganic acid, if necesary.

**6.** Method for preparing pharmaceutical compositions, characterized in that it consists in mixing an hexahydroazepin derivative of formula:

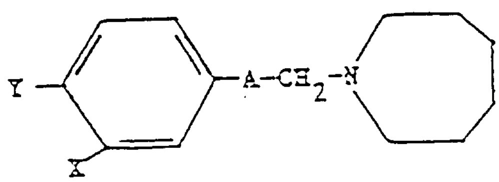

(I)

in which:

- A is a group selected from the following: $-CO-CH_2-$; $-CH(OH)-CH_2-$; -CH=CH-(cis); -C≡C-;
- X is hydrogen or a halogen atom;
- Y is a cyclohexyl group or when X is hydrogen, a phenyl group;

or one of its salts with organic or inorganic acids, with a pharmaceutical excipient.

7. Method according to claim 6, for the preparation of pharmaceutical compositions, in the form of unit doses.

8. Method according to claim 7, for the preparation of pharmaceutical compositions, containing from 0.5 to 1,000 mg of active principle of formula (I) per unit dose.

9. Use of at least one hexahydroazepin derivative of formula:

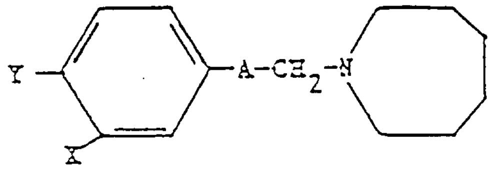

(I)

in which:

- A is a $-CH_2-CH_2-$ group or a -CH=CH-(trans) group,
- X is hydrogen or a halogen atom,
- Y is a cyclohexyl group or when X is hydrogen, a phenyl group;

or of at least one of its salts with organic or inorganic acids, for the preparation of pharmaceutical compositions useful for the treatment of psychotic disorders.

## Patentansprüche

**Patentansprüche für folgende Vertragsstaaten : DE, GB, FR, IT, NL, SE, CH, LI, BE, AT, LU, DK**

1. Hexahydroazepinderivate der Formel

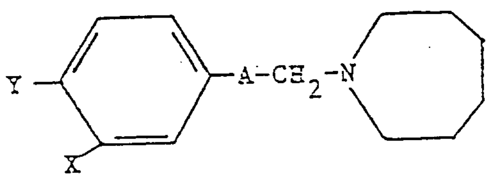

(I)

worin :

. A eine aus den folgenden : $-CO-CH_2-$; $-CH(OH)-CH_2-$; (Cis)-CH=CH-; -C≡C- gewählte Gruppe ist;
. X Wasserstoff oder ein Halogen darstellt;
. Y eine Cyclohexylgruppe oder wenn X Wasserstoff ist, eine Phenylgruppe ist; mit der Einschränkung, daß :
. wenn Y eine Cyclohexylgruppe und X Wasserstoff ist, A von $-CH(OH)-CH_2$-verschieden ist;
. wenn Y eine Phenylgruppe ist, A von $-CH(OH)-CH_2-$ oder $-CO-CH_2$-verschieden ist;

sowie ihre Salze mit Mineral- und organischen Säuren.

2. Pharmazeutisch akzeptable Additionssalze einer Verbindung nach Anspruch 1.

3. Hydrochlorid von 3-[Cis(Hexahydroazepin-1-yl)]-1- 3-(Chloro-4-Cyclohexyl Phenyl)-1-Propen.

4. Verfahren zur Herstellung eines Hexahydrozepin-Derivats nach Anspruch 1, der Formel (I), worin A aus den $-CO-CH_2-$ und $-CH(OH)-CH_2-$ Gruppen gewählt ist, dadurch gekennzeichnet, daß :

a) man eine Kondensationsreaktion mit Formaldehyd und Hexahydroazepin mit einem Acetophenon der Formel :

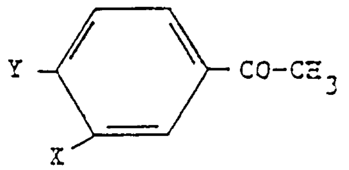

(II)

ausführt
worin X und Y die in Anspruch 1 angegebenen Bedeutungen haben, um einen Derivat der Formel (I), worin A die -CO-$CH_2$- Gruppe darstellt, zu erhalten;
b) man eventuell ein Reduktionsmittel mit der so erhaltenen Verbindung der Formel (I) reagieren läßt, um die Verbindung der Formel (I) herzustellen, worin A die -CH(OH)-$CH_2$-Gruppe darstellt;
c) man schließlich notwendigenfalls durch Zugabe einer geeigneten Mineral- order organischen Saüre ein Additionssalz einer so erhaltenen Verbindung der Formel (I) herstellt.

**5.** Verfahren zur Herstellung eines Hexahydroazepinderivats nach Anspruch 1, der Formel (I), worin A aus den -C≡C-, und (Cis)-CH=CH-Gruppen gewählt ist, dadurch gekennzeichnet, daß :

a) man eine Kondensationsreaktion mit Formaldehyd und Hexahydroazepin mit einem phenylacetylenischen Derivat der Formel :

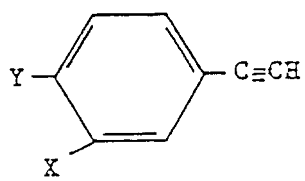

(III)

ausführt,
worin X und Y die in Anspruch 1 angegebenen Bedeutungen haben, um einen Derivat der Formel (I) zu erhalten, worin A die -C≡C- Gruppe darstellt ;
b) man eventuell, ausgehend von dem genannten Derivat, die vinylenische Verbindung der Formel (I) in Cis-Form herstellt, entweder direkt durch Hydrierung in Gegenwart eines metallischen Katalysators auf einem Träger, oder durch nicht selektive Hydrierung mit entspringenden Wasserstoff, mit nachfolgender Trennung der erhaltenen Mischung der Cis- und Trans-Isomere;
c) man schließlich notwendigenfalls durch Zugabe einer geeigneten Mineral- order organischen Saüre ein Additionssalz einer so erhaltenen Verbindung der Formel (I) herstellt.

**6.** Pharmazeutische Zusammensetzung, die als Wirkstoff einen Hexahydroazepinderivat der Formel :

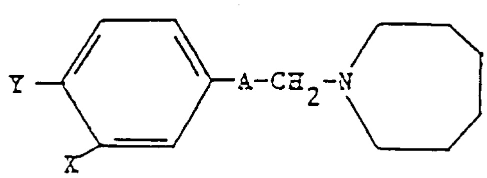

(I)

enthält,
worin A eine aus den folgenden : -CO-$CH_2$-; -CH(OH)-$CH_2$-; (Cis)-CH=CH-; -C≡C- gewählte Gruppe ist;

. X Wasserstoff oder ein Halogen darstellt;
. Y eine Cyclohexylgruppe oder wenn X Wasserstoff ist, eine Phenylgruppe ist;
oder eines seiner Salze mit Mineral- oder organischen Säuren.

7. Pharmazeutische Zusammensetzung nach Anspruch 6, in Form einer Dosierungseinheit, wobei der Wirkstoff mit einem pharmazeutischen Exzipienten vermischt ist.

8. Pharmazeutische Zusammensetzung nach Anspruch 7, die 0,5 bis 1000 mg Wirkstoff der Formel (I) pro Dosierungseinheit enthält.

9. Verwendung von mindestens einem Hexahydroazepinderivat der Formel:

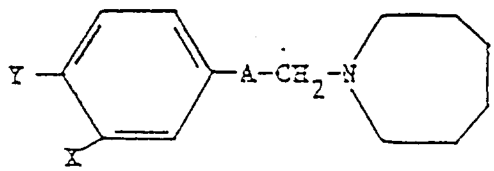

(I)

worin

. A eine $-CH_2-CH_2-$ Gruppe oder eine (Trans)-CH=CH- Gruppe darstellt,
. X Wasserstoff oder ein Halogen darstellt ;
. Y eine Cyclohexylgruppe oder wenn X Wasserstoff ist, eine Phenylgruppe ist ;
oder mindestens eines seiner Salze mit Mineral- oder organischen Säuren, für die Herstellung von pharmazeutischen Zusammensetzungen, die für die Behandlung psychotischer Störungen nützlich sind.

**Patentansprüche für folgenden Vertragsstaat : ES**

1. Verfahren zur Herstellung eines Hexahydroazepinderivats der Formel

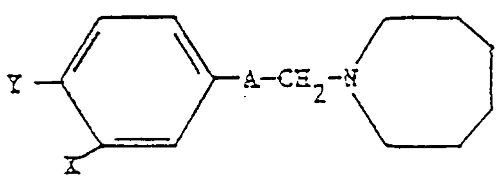

(I)

worin :

. A eine aus den folgenden : $-CO-CH_2-$; $-CH(OH)-CH_2-$ gewählte Gruppe ist;
. X Wasserstoff oder ein Halogen darstellt;
. Y eine Cyclohexylgruppe ist

mit der Einschränkung, daß, wenn X Wasserstoff ist, A von $-CH(OH)-CH_2-$verschieden ist;
sowie seinen Salze mit Mineral- und organischen Säuren,
dadurch gekennzeichnet, daß

a) man eine Kondensationsreaktion mit Formaldehyd und Hexahydroazepin mit einem Acetophenon der Formel :

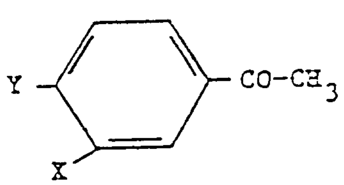

(II)

ausführt

worin X und Y die oben angegebenen Bedeutungen haben, um einen Derivat der Formel (I), worin A die -CO-$CH_2$-Gruppe darstellt, zu erhalten;

b) man eventuell ein Reduktionsmittel mit der so erhaltenen Verbindung der Formel (I) reagieren läßt, um die Verbindung der Formel (I) herzustellen, worin A die -CH(OH)-$CH_2$-Gruppe darstellt;

c) man schließlich notwendigenfalls durch Zugabe einer geeigneten Mineral- order organischen Saüre ein Additionssalz einer so erhaltenen Verbindung der Formel (I) herstellt.

2. Verfahren zur Herstellung eines Hexahydroazepinderivats der Formel :

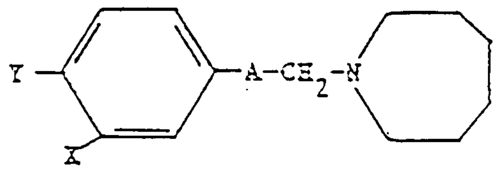

(I)

worin:

. A eine aus den folgenden :
(Cis)-CH=CH-;-C≡C- gewählte Gruppe ist;
. X Wasserstoff oder ein Halogen darstellt;
. Y eine Cyclohexylgruppe oder wenn X Wasserstoff ist, eine Phenylgruppe ist;

sowie seinen Salze mit Mineral- und organischen Säuren;
dadurch gekennzeichnet, daß :

a) man eine Kondensationsreaktion mit Formaldehyd und Hexahydroazepin mit einem phenylacetylenischen Derivat der Formel:

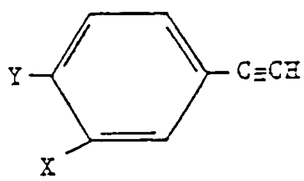

(III)

ausführt,

worin X und Y die oben angegebenen Bedeutungen haben, um einen Derivat der Formel (I) zu erhalten, worin A die -C=C- Gruppe darstellt ;

b) man eventuell , ausgehend von dem genannten Derivat, die vinylenische Verbindung der Formel (I) in Cis-Form herstellt, entweder direkt durch Hydrierung in Gegenwart eines metallischen Katalysators auf einem Träger, oder durch nicht selektive Hydrierung mit entspringenden Wasserstoff, mit nachfolgender Trennung der erhaltenen Mischung der Cis- und Trans-Isomere;

c) man schließlich notwendigenfalls durch Zugabe einer geeigneten Mineral- order organischen Saüre ein Additionssalz einer so erhaltenen Verbindung der Formel (I) herstellt.

3. Verfahren nach einem der Ansprüche 1 oder 2 zur Herstellung eines pharmazeutisch akzeptablen Additionssalzes einer Verbindung der Formel (I).

4. Verfahren nach Anspruch 2, dadurch gekennzeichnet, daß :

a) man eine Kondensationsreaktion von Formaldehyd und Hexahydroazepin mit einem phenylacetylenischen Derivat der Formel :

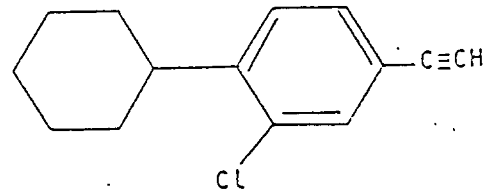

(III)

ausführt;

b) man eine Hydrierung der genannten Verbindung ausführt, um 3-(Cis(Hexahydroazepin-1-yl)) -1-(3-Chloro-4-Cyclohexyl Phenyl)-1-Propen ;

c) man die genannte Verbindung in Form von Hydrochlorid herstellt und abspaltet.

**5.** Verfahren zur Herstellung von pharmazeutischen Zusammensetzungen, dadurch gekennzeichnet, daß es darin besteht, einen Hexahydroazepinderivat der Formel :

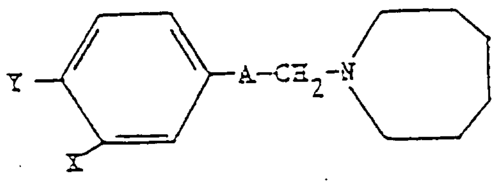

(I)

worin A eine aus den folgenden : $-CO-CH_2-$; $-CH(OH)-CH_2-$; (Cis)-CH=CH-;-C≡C- gewählte Gruppe ist;

. X Wasserstoff oder ein Halogen darstellt;
. Y eine Cyclohexylgruppe oder wenn X Wasserstoff ist, eine Phenylgruppe ist;

oder eines seiner Salze mit Mineral- oder organischen Säuren,
mit einem geeigneten Exzipienten zu Vermischen.

**6.** Verfahren nach Anspruch 5, zur Herstellung pharmazeutischer Zusammensetzungen, in Form einer Dosierungseinheit.

**7.** Verfahren nach Anspruch 6, zur Herstellung pharmazeutischer Zusammensetzungen, die 0,5 bis 1000 mg Wirkstoff der Formel (I) pro Dosierungseinheit enthalten.

**8.** Verwendung von mindestens einem Hexahydroazepinderivat der Formel :

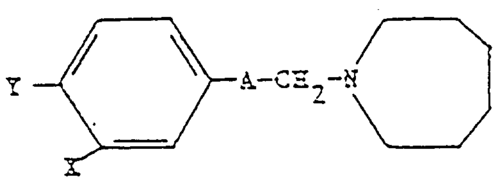

(I)

worin

. A eine $-CH_2-CH_2-$ Gruppe oder eine (Trans)-CH=CH- Gruppe darstellt,
. X Wasserstoff oder ein Halogen darstellt;
. Y eine Cyclohexylgruppe oder wenn X Wasserstoff ist, eine Phenylgruppe ist;

oder mindestens eines seiner Salze mit Mineral- oder organischen Säuren, für die Herstellung von pharmazeutischen Zusammensetzungen, die für die Behandlung psychotischer Störungen nützlich sind.

**Patentansprüche für folgenden Vertragsstaat : GR**

1. Hexahydroazepin derivate der Formel

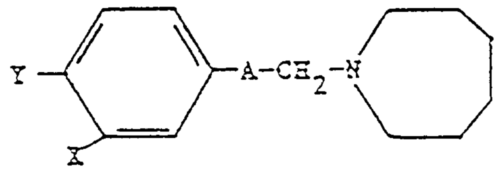

(I)

worin :

. A eine aus den folgenden : $-CO-CH_2-$; $-CH(OH)-CH_2-$; (Cis)-CH=CH-; -C≡C- gewählte Gruppe ist;
. X Wasserstoff oder ein Halogen darstellt;
. Y eine Cyclohexylgruppe oder wenn X Wasserstoff ist, eine Phenylgruppe ist; mit der Einschränkung, daß :
. wenn Y eine Cyclohexylgruppe und X Wasserstoff ist, A von $-CH(OH)-CH_2$ verschieden ist;
. wenn Y eine Phenylgruppe ist, A von $-CH(OH)-CH_2-$ oder $-CO-CH_2$-verschieden ist;

sowie ihre Salze mit Mineral- und organischen Säuren.

2. Pharmazeutisch akzeptable Additionssalze einer Verbindung nach Anspruch 1.

3. Hydrochlorid von 3-[Cis(Hexahydroazepin-1-yl)]-1-3-(Chloro-4-Cyclohexyl Phenyl)-1-Propen.

4. Verfahren zur Herstellung eines Hexahydrozepin-Derivats nach Anspruch 1, der Formel (I), worin A aus den $-CO-CH_2-$ und $-CH(OH)-CH_2-$ Gruppen gewählt ist, dadurch gekennzeichnet, daß :

   a) man eine Kondensationsreaktion mit Formaldehyd und Hexahydroazepin mit einem Acetophenon der Formel :

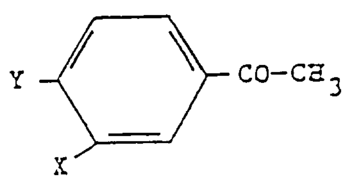

(II)

   ausführt
   worin X und Y die in Anspruch 1 angegebenen Bedeutungen haben, um einen Derivat der Formel (I), worin A die $-CO-CH_2$-Gruppe darstellt, zu erhalten;
   b) man eventuell ein Reduktionsmittel mit der so erhaltenen Verbindung der Formel (I) reagieren läßt, um die Verbindung der Formel (I) herzustellen, worin A die $-CH(OH)-CH_2$-Gruppe darstellt;
   c) man schließlich notwendigenfalls durch Zugabe einer geeigneten Mineral- order organischen Saüre ein Additionssalz einer so erhaltenen Verbindung der Formel (I) herstellt.

5. Verfahren zur Herstellung eines Hexahydroazepinderivats nach Anspruch 1, der Formel (I), worin A aus den -C≡C-, und (Cis)-CH=CH-Gruppen gewählt ist, dadurch gekennzeichnet, daß :

a) man eine Kondensationsreaktion mit Formaldehyd und Hexahydroazepin mit einem phenylacetylenischen Derivat der Formel :

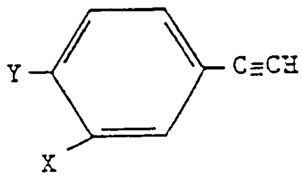

(III)

ausführt,
worin X und Y die in Anspruch 1 angegebenen Bedeutungen haben, um einen Derivat der Formel (I) zu erhalten, worin A die -C≡C- Gruppe darstellt ;

b) man eventuell, ausgehend von dem genannten Derivat, die vinylenische Verbindung der Formel (I) in Cis-Form herstellt, entweder direkt durch Hydrierung in Gegenwart eines metallischen Katalysators auf einem Träger, oder durch nicht selektive Hydrierung mit entspringenden Wasserstoff, mit nachfolgender Trennung der erhaltenen Mischung der Cis- und Trans-Isomere;

c) man schließlich notwendigenfalls durch Zugabe einer geeigneten Mineral- order organischen Saüre ein Additionssalz einer so erhaltenen Verbindung der Formel (I) herstellt.

6. Verfahren zur Herstellung von pharmazeutischen Zusammensetzungen, dadurch gekennzeichnet, daß es darin besteht, einen Hexahydroazepinderivat der Formel :

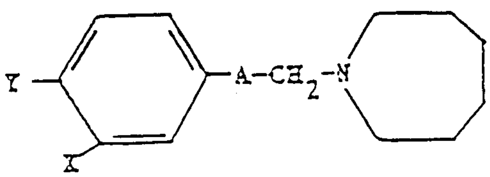

(I)

worin A eine aus den folgenden : $-CO-CH_2-$; $CH(OH)-CH_2-$; (Cis)-CH=CH-; -C≡C- gewählte Gruppe ist;

. X Wasserstoff oder ein Halogen darstellt;
. Y eine Cyclohexylgruppe oder wenn X Wasserstoff ist, eine Phenylgruppe ist;

oder eines seiner Salze mit Mineral- oder organischen Säuren,
mit einem pharmazeutischen Exzipienten zu vermischen.

7. Verfahren nach Anspruch 6, zur Herstellung pharmazeutischer Zusammensetzungen, in Form einer Dosierungseinheit.

8. Verfahren nach Anspruch 7, zur Herstellung pharmazeutischer Zusammensetzungen, die 0,5 bis 1000 mg Wirkstoff der Formel (I) pro Dosierungseinheit enthalten.

9. Verwendung von mindestens einem Hexahydroazepinderivat der Formel :

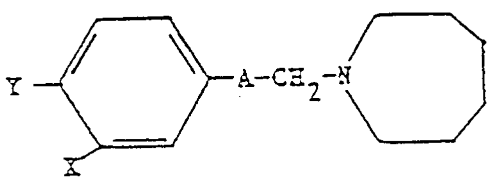

(I)

worin

- A eine $-CH_2-CH_2-$ Gruppe oder eine (Trans)-CH=CH- Gruppe darstellt,
- X Wasserstoff oder ein Halogen darstellt;
- Y eine Cyclohexylgruppe oder wenn X Wasserstoff ist, eine Phenylgruppe ist;

oder mindestens eines seiner Salze mit Mineral- oder organischen Säuren,
für die Herstellung von pharmazeutischen Zusammensetzungen, die für die Behandlung psychotischer Störungen nützlich sind.